# EUROPEAN PATENT SPECIFICATION

(11) **EP 3 131 453 B1**
(45) Date of publication and mention of the grant of the patent: **20.01.2021**
(21) Application number: 15780001.2
(22) Date of filing: 17.04.2015
(51) Int. Cl.: A61B 3/024, A61B 5/0478, A61B 5/0484, A61B 5/04, A61B 5/0496, A61B 5/00, A61B 5/16

(54) **PORTABLE BRAIN ACTIVITY SENSING PLATFORM FOR ASSESSMENT OF VISUAL FIELD DEFICITS**
TRAGBARE PLATTFORM ZUR MESSUNG DER GEHIRNAKTIVITÄT ZUR BEURTEILUNG VON SICHTFELDDEFIZITEN
PLATE-FORME PORTABLE DE DÉTECTION DE L'ACTIVITÉ DU CERVEAU POUR L'ÉVALUATION DE DÉFICITS DE CHAMP VISUEL

(30) Priority: 17.04.2014 US 201461981145 P
(43) Date of publication of application: 22.02.2017
(73) Proprietor: The Regents of the University of California, Oakland, CA 94607-5200 (US)
(72) Inventor: JUNG, Tzyy-Ping, San Diego, CA 92130 (US); LIN, Yuan-Pin, San Diego, CA 92122 (US); WANG, Yijun, San Diego, CA 92122 (US); MEDEIROS, Felipe, A., San Diego, CA 92127 (US)
(74) Representative: Potter Clarkson
(86) International application number: PCT/US2015/026543
(87) International publication number: WO 2015/161300

(56) References cited:
- WO-A1-01/78586
- WO-A1-2013/037050
- WO-A2-2007/026368
- US-A- 4 561 448
- US-A1- 2009 091 706
- US-A1- 2009 091 706
- US-A1- 2010 094 702
- BARLOW J S ET AL: "Eye movement artifact nulling in EEGs by multichannel on-line EOG subtraction", ELECTROENCEPHALOGRAPHY AND CLINICAL NEUROPHYSIOLOGY, ELSEVIER, AMSTERDAM, NL, vol. 52, no. 5, 1 November 1981 (1981-11-01), pages 418-423, XP024292229, ISSN: 0013-4694, DOI: 10.1016/0013-4694(81)90024-9 [retrieved on 1981-11-01]
- CHRISTOPH S. HERRMANN: "Human EEG responses to 1-100 Hz flicker: resonance phenomena in visual cortex and their potential correlation to cognitive phenomena", EXPERIMENTAL BRAIN RESEARCH., vol. 137, no. 3-4, 2 April 2001 (2001-04-02), pages 346-353, XP055421388, DE ISSN: 0014-4819, DOI: 10.1007/s002210100682
- MARTIN OEHLER ET AL: "Extraction of SSVEP signals of a capacitive EEG helmet for Human Machine Interface", ENGINEERING IN MEDICINE AND BIOLOGY SOCIETY, 2008. EMBS 2008. 30TH ANNUAL INTERNATIONAL CONFERENCE OF THE IEEE, 1 August 2008 (2008-08-01), pages 4495-4498, XP055421230, Piscataway, NJ, USA DOI: 10.1109/IEMBS.2008.4650211 ISBN: 978-1-4244-1814-5
- NAVEEN K. YADAV ET AL: "Effect of different stimulus configurations on the visual evoked potential (VEP)", DOCUMENTA OPHTHALMOLOGICA., vol. 124, no. 3, 1 June 2012 (2012-06-01), pages 177-196, XP055421246, NL ISSN: 0012-4486, DOI: 10.1007/s10633-012-9319-0
- LIN, YP ET AL.: 'Assessing the quality of steady-state visual-evoked potentials for moving humans using a mobile electroencephalogram headset.' FRONTIERS IN HUMAN NEUROSCIENCE. vol. 8, 31 March 2014, pages 1 - 10, XP055359502
- LIN, YP ET AL.: 'Using Multifocal Steady-State Visual Evoked Potentials for Objective Assessment of Visual Field Loss: A Pilot Study.' INVESTIGATIVE OPHTHALMOLOGY & VISUAL SCIENCE . vol. 55, no. ISSUE, April 2014, page 5126, XP055376533

## Description

### TECHNICAL FIELD

This patent document relates to systems, devices, and processes that use brain machine interface (BMI) technologies.

### BACKGROUND

Electroencephalography (EEG) is the recording of electrical activity exhibited by the brain using electrodes positioned on a subject's scalp, forming a spectral content of neural signal oscillations that comprise an EEG data set. For example, the electrical activity of the brain that is detected by EEG techniques can include voltage fluctuations, e.g., resulting from ionic current flows within the neurons of the brain. In some contexts, EEG refers to the recording of the brain's spontaneous electrical activity over a short period of time, e.g., less than an hour. EEG can be used in clinical diagnostic applications including epilepsy, coma, encephalopathies, brain death, and other diseases and defects, as well as in studies of sleep and sleep disorders. In some instances, EEG has been used for the diagnosis of tumors, stroke and other focal brain disorders. US 2009/0091706 describes a method for determining a likelihood of a visual deficit in a subject using a simultaneously multi-temporal visual test. At least two visual patterns are simultaneously displayed to the subject. Each pattern reverses in contrast or color at a different display frequency, and each pattern is displayed to a different region of the subject's visual field. Electrical activity of the brain of the subject is captured and sampled, and one or more frequency components are resolved from the resulting signal, where each frequency component corresponds to a different display frequency. The method then involves determining from the frequency components, optionally by comparison between the eyes, a measurement of a likelihood that a visual deficit exists in a particular area. J S Barlow and A Rémond: Eye Movement Artifact Nulling In EEGs by Multichannel On-Line EOG subtraction; Electroencephalography and Clinical Neurophysiology, 1981, 52: 418-423 describes a multichannel nulling or subtraction technique for minimizing or eliminating vertical and/or horizontal eye movement artifact including blinks in EEG recordings. Document WO01/78586A1 discloses the use of glasses or goggles in the context of the assessment of mfVEP using portable systems. D7 discloses "dartboard patterns" used as visula stimuli, but D7 is silent about concurrent (simultaneous) presentation of all the flickering regions at different frequencies and the ensuing evoked mfSSVEP.

### SUMMARY

Disclosed are electroencephalogram (EEG)-based brain sensing methods, systems, and devices for visual-field examination by using high-density EEG to associate the dynamics of multifocal steady-state visual-evoked potentials (mfSSVEP) with visual field defects. In some aspects, the disclosed techniques integrate mfSSVEPs into a portable platform using wireless EEG and a head mounted display, demonstrating ability to assess potential visual field deficits, e.g., in conditions such as glaucoma.

In one aspect, a system for monitoring brain activity associated with visual field of a user includes a sensor unit to acquire electroencephalogram (EEG) signals including one or more electrodes attached to a casing wearable on the head of a user; visual display unit including a display screen to present visual stimuli to the user in a plurality of sectors of a visual field, in which the presented visual stimuli includes an optical flickering effect at a selected frequency mapped to each sector of the visual field, the visual stimuli configured to evoke multifocal steady-state visual-evoked potentials (mfSSVEP) in the EEG signals exhibited by the user acquired by the sensor unit; and a data processing unit in communication with the sensor unit and the visual display unit to analyze the acquired EEG signals and produce an assessment of the user's visual field.

In one aspect, a method for examining a visual field of a subject includes presenting, to a subject, visual stimuli in a plurality of sectors of a visual field of a subject, in which for each sector the presented visual stimuli includes an optical flickering effect at a selected frequency; acquiring electroencephalogram (EEG) signals from one or more electrodes in contact with the head of the subject; processing the acquired EEG signals to extract multifocal steady-state visual-evoked potentials (mfSSVEP) data associated with the subject's EEG signal response to the presented visual stimuli; and producing a quantitative assessment of the visual field of the subject based on the MfSSVEP data.

In one aspect, a portable system for monitoring brain activity associated with visual field of a user includes a brain signal sensor device to acquire electroencephalogram (EEG) signals including one or more electrodes attached to a casing wearable on the head of a user; a wearable visual display unit to present visual stimuli to the user and structured to include a display screen and a casing able to secure to the head of the user, in which the wearable visual display is operable to present the visual stimuli in a plurality of sectors of the user's visual field, such that for each sector the presented visual stimuli includes an optical flickering effect at a selected frequency, and in which the visual stimuli are configured to evoke multifocal steady-state visual-evoked potentials (mfSSVEP) in the EEG signals exhibited by the user acquired by the brain signal sensor device; a data processing unit in communication with the brain signal sensor device and the wearable visual display unit to provide the visual stimuli to the wearable visual display unit and to analyze the acquired EEG signals and produce an assessment of the user's visual field; and an electrooculogram (EOG) unit including one or more electrodes to be placed proximate the outer canthus of each of the user's eyes to measure corneo-retinal standing potential (CRSP) signals, in which the one or more electrodes of the EOG unit are in communication with the data processing unit to process the acquired CRSP signals from the one or more electrodes to determine movements of the user's eyes.

The subject matter described in this patent document can be implemented in specific ways that provide one or more of the following features. For example, by removing the subjectivity inherent to standard perimetry, the disclosed portable platform for assessing functional loss can facilitate detection and monitoring of visual field loss in glaucoma, the leading cause of blindness in the world. The disclosed portable platform uses high-density EEG recording and mfSSVEP that can provide improved signal-to-noise ratios, increasing reproducibility and diagnostic accuracy, e.g., as compared to existing EEG-based methods for objective perimetry, such as mfVEP. As a portable platform that could be used for testing in unconstrained situations, the disclosed methods can allow for much broader and more frequent testing of patients, e.g., as compared to existing perimetric approaches. For example, this could reduce the number of office visits necessary for patients at risk or diagnosed with glaucoma, significantly decreasing the economic burden of the disease. In addition, by allowing more frequent testing, the disclosed methods can facilitate the discrimination of true deterioration from test-retest variability, e.g., resulting in earlier diagnosis and detection of progression. The disclosed portably-implemented and objective methods for visual field assessment can also allow screening for visual loss in underserved populations. The disclosed technology is non-invasive and can be implemented without direct physical contact with an eye of the individual subject being assessed, and thereby avoid causing any discomfort or risk of injury through inadvertent application of force or transfer of harmful chemical or biological material to the eye.

Those and other features are described in greater detail in the drawings, the description and the claims.

### BRIEF DESCRIPTION OF THE DRAWINGS

FIG. 1A shows a diagram of an exemplary system to implement the techniques of the disclosed technology.
FIGS. IB-IE show diagrams of an exemplary method to examine visual field defects using the disclosed technology.
FIG. IF shows diagrams depicting an exemplary implementation using multifocal steady-state visual-evoked potentials (mfSSVEP) for assessment of visual field defects.
FIG. 2 shows images of an exemplary EEG setup used in exemplary implementations of the disclosed techniques.
FIG. 3A shows data plots depicting exemplary CONTROL-DEFICIT comparisons, in which the exemplary data represents the SSVEP frequency with the lowest signal-to-noise-ratio versus other frequencies.
FIG. 3B shows data plots depicting a BLOCK-CONTROL comparison in mfSSVEP profiles from a representative subject.
FIG. 4 shows an image of an exemplary portable device of the disclosed portable, objective mfSSVEP-based visual field assessment platform.
FIGS. 5A and 5B show images of an exemplary wearable, wireless 64-channel dry EEG system.
FIGS. 6A and 6B show an image and a diagram of an exemplary head-mounted display system and presentation layout to present an exemplary mfSSVEP stimulation, respectively.

### DETAILED DESCRIPTION

Optic neuropathy refers to optic nerve damage that can result in significant and irreversible loss of visual function and disability. One example is glaucoma. Glaucoma is associated with a progressive degeneration of retinal ganglion cells (RGCs) and their axons, resulting in a characteristic appearance of the optic disc and a concomitant pattern of visual field loss. Loss of visual function in glaucoma is generally irreversible, and without adequate treatment the disease can progress to disability and blindness. The disease can remain relatively asymptomatic until late stages and, therefore, early detection and monitoring of functional damage is paramount to prevent functional impairment and blindness.

It is estimated that glaucoma affects more than 70 million individuals worldwide with approximately 10% being bilaterally blind, which makes it the leading cause of irreversible blindness in the world. However, as the disease can remain asymptomatic until it is severe, the number of affected individuals is likely to be much larger than the number known to have it. Population-level survey data indicate that only 10% to 50% of the individuals are aware they have glaucoma.

Visual dysfunction appears to be a strong predictor of cognitive dysfunction in subject in a number of clinical neuroscience disorders. For example, the functional deficits of glaucoma and Alzheimer's Disease include loss in low spatial frequency ranges in contrast sensitivity, and are similar in both diseases. Pattern masking has been found to be a good predictor of cognitive performance in numerous standard cognitive tests. Some tests found to correlate with pattern masking include Gollin, Stroop-Work, WAIS-PA, Stroop-Color, Geo-Complex Copy, Stroop-Mixed and RCPM, for example. Losses in contrast sensitivity at the lowest spatial frequency also was predictive of cognitive losses in the seven tests. For example, AD subjects have abnormal word reading thresholds corresponding to their severity of cognitive impairment and reduced contrast sensitivity in all spatial frequencies as compared to normal subjects.

Assessment of functional loss in the disease has traditionally been made using standard automated perimetry (SAP). SAP is the current standard for assessment of visual field loss. Visual field assessment with SAP requires considerable subjective input from the patient, and for some patients it is very difficult, or even impossible, to obtain reliable visual field measures. SAP is also limited by large test-retest variability and a large number of tests are usually necessary in order to discriminate true disease progression from noise, even for reliable test-takers. For example, SAP testing is limited by subjectivity of patient responses and large variability, frequently requiring a large number of tests for effective detection of change over time. These tests are generally conducted in clinic-based settings and, due to limited patient availability and health care resources, an insufficient number of tests are frequently acquired over time, resulting in delayed diagnosis and detection of disease progression. The requirements for highly trained technicians, cost, complexity, and lack of portability of SAP testing also preclude its use for screening of visual field loss in underserved populations. For example, because SAP, testing is generally performed in a clinic-based setting and requires highly trained technicians, this limits the availability of this testing resource, which frequently results in patients not undergoing the necessary number of tests to allow detection of disease progression over time, and thereby resulting in late diagnosis or delayed detection of progression. Perimeters are also usually expensive and not easily transportable, which has largely impeded the use of the SAP technique for screening or assessment of visual field loss in remote settings and in underserved populations. Neurological disorders, e.g., such as macular degeneration, diabetic retinopathy, optic neuritis, papilledema, anterior ischemic optic neuropathy, and tumor, can be diagnosed and tracked by SAP.

Objective assessment of visual field damage in glaucoma has been attempted with the use of visual evoked potential (VEP) and multifocal VEP techniques. However, these VEP techniques have been limited by relatively low signal to noise ratio and have shown limited potential to assess visual field losses in the disease. For example, the conventional pattern VEP is predominantly generated by cortical elements receiving projections from the central retina, where the central 2° of visual field contributes 65% of the response. Therefore, conventional VEP possesses a limited ability to reflect field loss in non-central areas, such as those that can occur with glaucoma. While multifocal VEP techniques can allow many areas of the retina to be stimulated simultaneously and separate responses from each part of the visual field to be obtained, individual differences in the anatomy of the visual cortex lead to considerable inter-individual variability of responses in normal subjects, making it difficult the identification of mfVEP abnormalities in diseased patients. Further, existing mfVEP recording techniques can only be performed with non-portable devices in clinic- or laboratory-based settings, requiring cumbersome setup for placement of electrodes (e.g., data collection requires skin preparation and gel application to ensure good electrical conductivity between sensor and skin), and such procedures are time consuming and uncomfortable for the patient.

The present technology includes methods, systems, and devices that acquire, process, and/or utilize steady-state visually evoked potentials (SSVEP) for monitoring, tracking, and/or diagnosing various paradigms in cognitive function (e.g., visual attention, binocular rivalry, working memory, and brain rhythms) and clinical neuroscience (e.g., aging, neurodegenerative disorders, schizophrenia, ophthalmic pathologies, migraine, autism, depression, anxiety, stress, and epilepsy), particularly for SSVEPs generated by optical stimuli.

Disclosed are electroencephalogram (EEG)-based brain sensing methods, systems, and devices for visual-field examination by using high-density EEG to associate the dynamics of multifocal steady-state visual-evoked potentials (mfSSVEP) with visual field defects. In some aspects, the disclosed techniques integrate mfSSVEPs into a portable platform using wireless EEG and a head mounted display capable of assessing potential visual field deficits. In some examples that are not part of the invention, the disclosed technology can be applied to diagnose and track neurological disorders, e.g., such as macular degeneration, diabetic retinopathy, optic neuritis, papilledema, anterior ischemic optic neuropathy, and/or tumors.

For example, in contrast to the transient event-related potentials elicited during a conventional VEP or mfVEP examination, the present technology utilizes rapid flickering stimulation to produce a brain response characterized by a "quasi-sinusoidal" waveform whose frequency components are constant in amplitude and phase, e.g., the so-called steady-state response. In some embodiments, for example, the portable platform integrates a wearable, wireless, high-density dry EEG system and a head-mounted display allowing users to routinely monitor the electrical brain activity associated with visual field stimulation. The present technology includes brain-computer interfaces using dry EEG sensor arrays, wearable/wireless data acquisition and signal processing hardware and software. These interfaces can monitor and record non-invasive, high spatiotemporal resolution brain activity of unconstrained, actively engaged human subjects.

For example, the disclosed technology can be applied for ophthalmologic diagnosis of neurological complications, in particular that of major ocular pathologies including glaucoma, retinal anomalies and of sight, retinal degeneration of the retinal structure and macular degeneration, diabetic retinopathy, optic neuritis, optical neuroma, or degenerative diseases, e.g., such as Parkinson's disease, Alzheimer's disease, non-Alzheimer's dementia, multiple sclerosis, ALS, head trauma, diabetes, or other cognitive disorders, e.g., such as dyslexia. More broadly, the present technology can be used to characterize inappropriate responses to contrast sensitivity patterns, and disorders affecting the optical nerve and the visual cortex.

Similarly, as an example that is not part of the invention, the disclosed technology can be used for multiple sclerosis (MS). It is known that MS affects neurons and that the effect comes and goes with time. There is apparent recovery of the cells at least in early stages of the disease. One would therefore expect the diagnosed areas of loss in the visual field to move around the visual field over time, and perhaps to recovery temporarily. As the disease progresses to the point where there is a lot of loss on the retina, the areas of loss will remain lost and will not show temporary recovery. The retina and brain do parallel processing to determine relative position of adjacent objects. In the case of dyslexia, for example, this processing somehow gets reversed and the subject mixes up the order of letters in words or even the order of entire words. This too could show up as an apparent ganglion cell loss. Again, the apparent loss could be from the ganglion cells or from the feedback to the lateral geniculate nucleus. The disclosed technology includes portable platforms that can be used for accurate and convenient screening of many neuro-degenerative diseases, e.g., including Alzheimer's, non-Alzheimer's dementia, Parkinson's, multiple sclerosis, macular degeneration, ALS, diabetes, dyslexia, head trauma, and others.

The present technology utilizes electroencephalogram (EEG)-based brain sensing methods, systems, and devices for visual-field examination by using high-density EEG to associate the dynamics of multifocal steady-state visual-evoked potentials (mfSSVEP) with visual field defects, in which the use of rapid flickering stimulation can produce a brain response characterized by a "quasi-sinusoidal" waveform whose frequency components are constant in amplitude and phase, the so-called steady-state response. Steady-state VEPs have desirable properties for use in the assessment of the integrity of the visual system. For example, the disclosed techniques are faster than mfVEP, less susceptible to artifacts produced by blinks and eye movements, to electromyographic noise contamination and may present better signal to noise (SNR) ratio. mfSSVEP is a form of steady-state visual-evoked potentials which reflect a frequency-tagged oscillatory EEG activity modulated by the frequency of periodic visual simulation higher than 6 Hz. Different from the ordinary SSVEP, the mfSSVEP is a signal of multi-frequency tagged SSVEP, e.g., which can be elicited by simultaneously presenting multiple continuous, repetitive black/white reversing visual patches flickering at different frequencies. Based on the nature of mfSSVEP, a flicker sector(s) corresponding to a visual field deficit(s) will be less perceivable or unperceivable and thereby will elicit a weaker SSVEP, e.g., as compared to the brain responses to other visual stimuli presented at normal visual spots.

The disclosed methods, systems, and devices for visual-field examination using mfSSVEP data includes the formation of a spatial visual stimulus display having multiple regions or sectors at different spatial locations, where for each region, the particular region includes an optical effect (e.g., light flickering) that changes at a unique frequency with respect to at least a proximate region or any other region of the visual stimulus display. For example, the visual stimulus display can include twenty regions each presenting its respective optical effect at a different frequency between 8.0 Hz and 11.8 Hz (e.g., 8.0 in sector 1, 8.2 in sector 2, 8.4 in sector 3, ..., 11.8 in sector 20). For example, the optical effect can be a light modulating between an ON and OFF state at the designated frequency for the particular region. The brain response to the visual stimuli, e.g., as measured by at least one electrode placed on or near the head or multiple electrodes arranged in an arrangement to improve spatiotemporal resolution of the EEG signals, are acquired and processed to produce and evaluate mfSSVEP data with respect to a frequency spectrum including the designated frequencies mapped to the spatial regions of the visual stimulus display. The processing includes comparing the mfSSVEP signal at the particular frequencies to a predetermined threshold, or in relation to other mfSSVEP signals, to determine if the signal falls below the predetermined threshold or is substantially lower with respect to a comparative mfSSVEP signal. Determination of a mfSSVEP signal below the threshold or substantially lower than a standard at that particular frequency corresponds to a visual field deficiency to the spatial location on the visual stimulus display to which that frequency is mapped, and indicative of a visual field defect of the user in that specific region.

In some examples that are not part of the invention, the disclosed techniques include using SSVEP and brain-computer interfaces (BCIs) to bridge the human brain with computers or external devices. By detecting the SSVEP frequencies from the non-invasively recorded EEG, the users of SSVEP-based brain-computer interface can interact with or control external devices and/or environments through gazing at distinct frequency-coded targets. For example, the SSVEP-based BCI can provide a promising communication carrier for patients with disabilities due to its high signal-to-noise ratio over the visual cortex, which can be measured by EEG at the parieto-occipital region noninvasively. Methods, devices and systems of the disclosed technology can implement wireless SSVEP data acquisition and processing. Methods, devices and systems of the disclosed technology can include a noninvasive platform for continuously monitoring high temporal resolution brain dynamics without requiring conductive gels applied to the scalp. An exemplary system can employ dry microelectromechanical system EEG sensors, low-power signal acquisition, amplification and digitization, wireless telemetry, and real-time processing. In addition, the present technology can include analytical techniques, such as independent component analysis, which can improve detectability of SSVEP signals.

Exemplary implementations of the disclosed multifocal techniques to SSVEP (mfSSVEP) are described in this patent document. The exemplary results of such implementations demonstrate detection of localized and peripheral field losses, as well as show successful implementations of a portable objective method of assessment of visual field loss in opto-neurological diseases, e.g., such as glaucoma, amblyopia, age-related macular degeneration, and optic neuritis.

Diagnosis and detection of progression of neurological disorders remain challenging tasks. For example, a validated portable objective method for assessment of visual field loss would have numerous advantages compared to currently existing methods to assess functional loss in the disease. An objective EEG-based test would remove the subjectivity and decision-making involved when performing perimetry, potentially improving reliability of the test. A portable and objective test could be done quickly at home under unconstrained situations, decreasing the required number of office visits and the economic burden of the disease. In addition, a much larger number of tests could be obtained over time. This would greatly enhance the ability of separating true deterioration from measurement variability, potentially allowing more accurate and earlier detection of progression. In addition, more precise estimates of rates of progression could be obtained. Even if the spatial resolution of mfSSVEP does not provide a higher resolution than SAP techniques, the increased number of tests available for analysis over time can still provide more reliable assessment of visual field defects. The exemplary visual field assessment methods can be used for screening in remote locations or for monitoring patients with the disease in underserved areas, as well as for use in the assessment of visual field deficits in other conditions.

There are few if any currently available reliable and effective portable methods for assessment of functional loss in such disorders. The disclosed technology includes a portable platform that integrates a wearable, wireless EEG dry system and a head-mounted display system that allows users to routinely and continuously monitor the electrical brain activity associated with visual field in their living environments, e.g., representing a transformative way of monitoring disease progression, e.g., such as in glaucoma. In addition, such devices provide an innovative and potentially useful way of screening for the disease. The disclosed technology includes portable brain-computer interfaces and methods for sophisticated analysis of EEG data, e.g., including capabilities for diagnosis and detection of disease progression. For example, the disclosed methods, systems, and devices can be implemented to improve screening, diagnosis and detection of disease progression and also enhance understanding of how the disease affects the visual pathways.

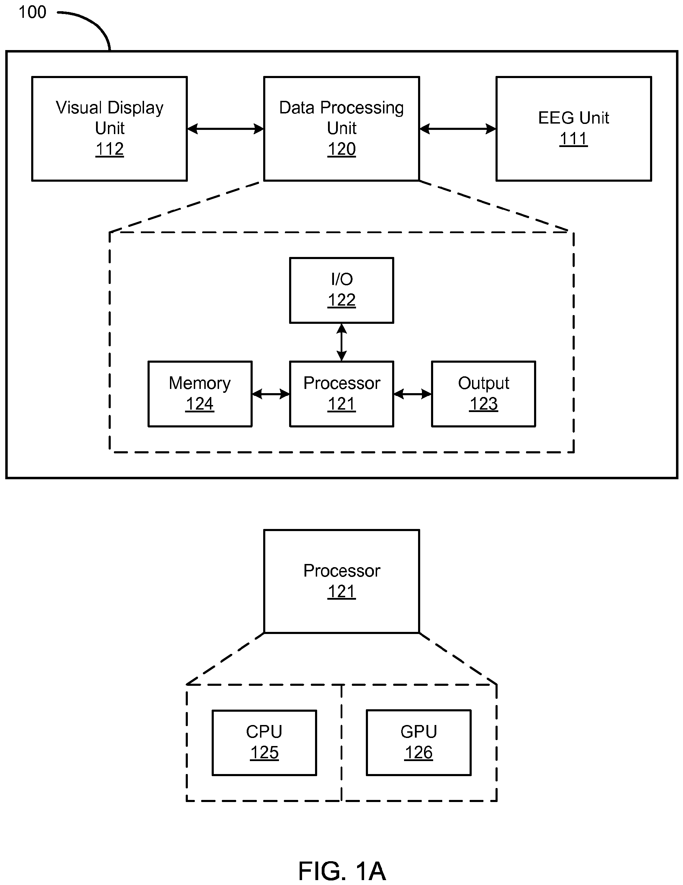

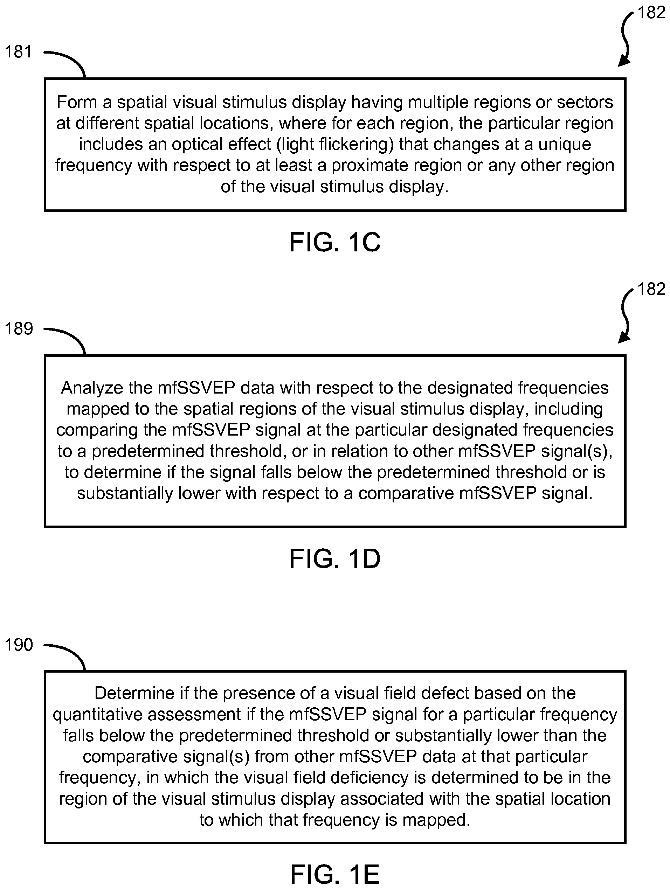

FIG. 1A shows a diagram of an exemplary portable EEG-based system 100 of the disclosed technology to implement the methods described in this patent document. The EEG-based system 100 integrates a wearable, wireless, high-density dry EEG sensor unit 111 and a visual display unit 112 (e.g., such as a head-mounted display) in data communication with a data processing unit 120 allowing users to routinely monitor the electrical brain activity associated with visual field stimulation. The data processing unit 120 can include various modules or units of the disclosed system for processing data extracted from the subject, e.g., via the EEG unit 111, based on stimulus of the subject via the visual display unit 112.

The visual display unit 112 can include an output unit that can include various types of display, speaker, and/or printing interfaces, e.g., which can be used to implement a visual stimulus technique. For example, the output unit can include cathode ray tube (CRT), light emitting diode (LED), or liquid crystal display (LCD) monitor or screen, among other visual displays, as a visual display. In some examples, the output unit can include various types of audio signal transducer apparatuses or other sensory inducing apparatuses to implement the sensory stimuli.

The data processing unit 120 can include a processor 121 that can be in communication with an input/output (I/O) unit 122, an output unit 123, and a memory unit 124. The data processing unit 120 can be implemented as one of various data processing systems, such as a personal computer (PC), laptop, and mobile communication device. In some implementations, the data processing unit 120 can be included in the device structure that includes the wearable EEG sensor unit 111. To support various functions of the data processing unit 120, the processor 121 can be included to interface with and control operations of other components of the data processing unit 120, such as the I/O unit 122, the output unit 123, and the memory unit 124.

The memory unit 124 can store information and data, e.g., such as instructions, software, values, images, and other data processed or referenced by the processor 121. Various types of Random Access Memory (RAM) devices, Read Only Memory (ROM) devices, Flash Memory devices, and other suitable storage media can be used to implement storage functions of the memory unit 124. The memory unit 124 can store data and information, which can include subject stimulus and response data, and information about other units of the system, e.g., including the EEG sensor unit 111 and the visual display unit 112, such as device system parameters and hardware constraints. The memory unit 124 can store data and information that can be used to implement the portable EEG-based system 100.

The I/O unit 122 can be connected to an external interface, source of data storage, or display device. Various types of wired or wireless interfaces compatible with typical data communication standards can be used in communications of the data processing unit 120 with the EEG sensor unit 111 and the visual display unit 112 and/or other units of the system, e.g., including, but not limited to, Universal Serial Bus (USB), IEEE 1394 (FireWire), Bluetooth, IEEE 802.111, Wireless Local Area Network (WLAN), Wireless Personal Area Network (WPAN), Wireless Wide Area Network (WWAN), WiMAX, IEEE 802.16 (Worldwide Interoperability for Microwave Access (WiMAX)), 3G/4G/LTE cellular communication methods, and parallel interfaces, can be used to implement the I/O unit 122. The I/O unit 122 can interface with an external interface, source of data storage, or display device to retrieve and transfer data and information that can be processed by the processor 121, stored in the memory unit 124, or exhibited on the output unit 123.

In some implementations of the system 100, the data processing unit 120 can include an output unit 123 that can be used to exhibit data implemented by the data processing unit 120. The output unit 123 can include various types of display, speaker, or printing interfaces to implement the output unit 123. For example, the output unit 123 can include cathode ray tube (CRT), light emitting diode (LED), or liquid crystal display (LCD) monitor or screen as a visual display to implement the output unit 123. In other examples, the output unit 123 can include toner, liquid inkjet, solid ink, dye sublimation, inkless (e.g., such as thermal or UV) printing apparatuses to implement the output unit 123; the output unit 123 can include various types of audio signal transducer apparatuses to implement the output unit 123. The output unit 123 can exhibit data and information, such as the system data in a completely processed or partially processed form. The output unit 123 can store data and information used to implement the disclosed techniques.

FIG. 1A also shows a block diagram of the processor 121 that can include a central processing unit (CPU) 125 and/or a graphic processing unit (GPU) 126, or both the CPU 125 and the GPU 126. The CPU 125 and GPU 126 can interface with and control operations of other components of the data processing unit 120, such as the I/O unit 122, the output unit 123, and the memory unit 124.

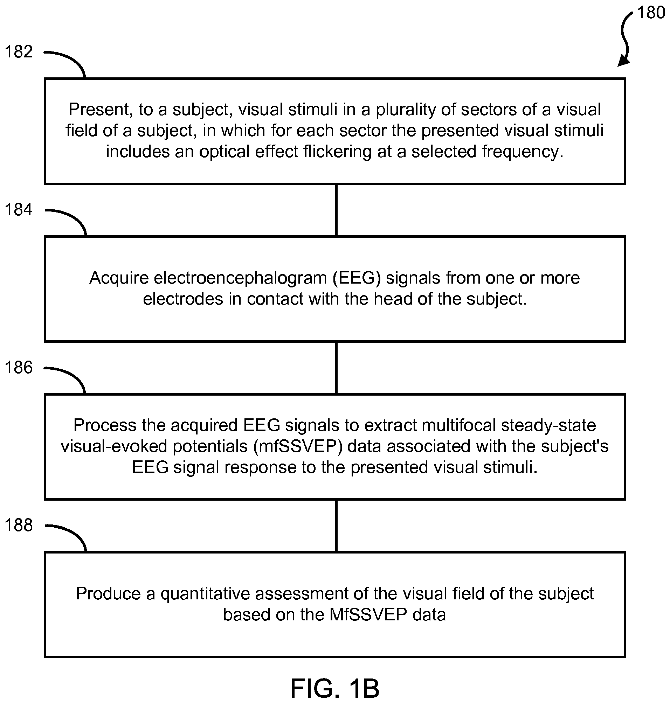

FIG. 1B shows a diagram of an exemplary method 180 to examine visual field defects using the disclosed technology, e.g., such as including the system 100. The method 180 includes a process 182 to present, to a subject, visual stimuli in a plurality of sectors of a visual field of a subject, in which for each sector the presented visual stimuli includes an optical effect (e.g., light flickering) at a selected frequency. The method 180 includes a process 184 to acquire EEG signals from one or more electrodes in contact with the head of the subject. The method 180 includes a process 186 to data process (e.g., analyze) the acquired EEG signals to extract mfSSVEP data associated with the subject's EEG signal response to the presented visual stimuli. The method 180 includes a process 188 to produce a quantitative assessment of the visual field of the subject based on the MfSSVEP data.

In some implementations of the method 180, for example, the quantitative assessment produced by the process 188 can provide an indication if there is a presence of a visual field defect in the user's visual field. In some implementations, for example, the process 188 can include a process to determine the presence of the visual field defect in a sector having a mfSSVEP signal below a predetermined threshold. In some implementations, for example, the visual stimuli presented by the process 182 can include multiple and repetitive optical effects flickering at the selected frequency in the corresponding sector of the visual field of the subject.

In some implementations of the method 180, for example, as shown in FIG. 1C, the process 182 includes a process 181 to provide the visual stimuli to the visual display unit 112 (e.g., including a wearable visual display unit) from the data processing unit 120, in which the providing can include generating the visual stimuli (e.g., produce and/or assign an optical flickering effect of the visual stimuli at a selected frequency associated with each sector of the visual field); and/or supplying a previously generated visual stimuli. In some implementations of the process 181, the process 181 to provide the visual stimuli includes forming a spatial visual stimulus display having multiple regions or sectors at different spatial locations, where for each region, the particular region includes an optical effect (e.g., light flickering) that changes at a unique frequency with respect to at least a proximate region or any other region of the visual stimulus display.

In some implementations of the method 180, for example, as shown in FIG. ID, the process 188 can include a process 189 to analyze the mfSSVEP data with respect to a frequency spectrum including the designated frequencies mapped to the spatial regions of the visual stimulus display, in which the analyzing can include comparing the mfSSVEP signal at the particular frequencies to a predetermined threshold, or in relation to another mfSSVEP signal or other mfSSVEPs (e.g., including from an averaged population or individual group of mfSSVEP data with respect to that particular frequency), to determine if the signal falls below the predetermined threshold or is substantially lower with respect to a comparative mfSSVEP signal.

In some implementations of the method 180, for example, as shown in FIG. 1E, the method can include a process 190 to determine if the presence of a visual field defect based on the quantitative assessment, e.g., if the mfSSVEP signal for a particular frequency falls below the predetermined threshold or substantially lower than the comparative signal(s) from other mfSSVEP data at that particular frequency, in which the visual field deficiency is determined to be in the region of the visual stimulus display associated with the spatial location to which that frequency is mapped.

### Exemplary Implementations of mfSSVEP Techniques for Assessment of Visual Field Loss

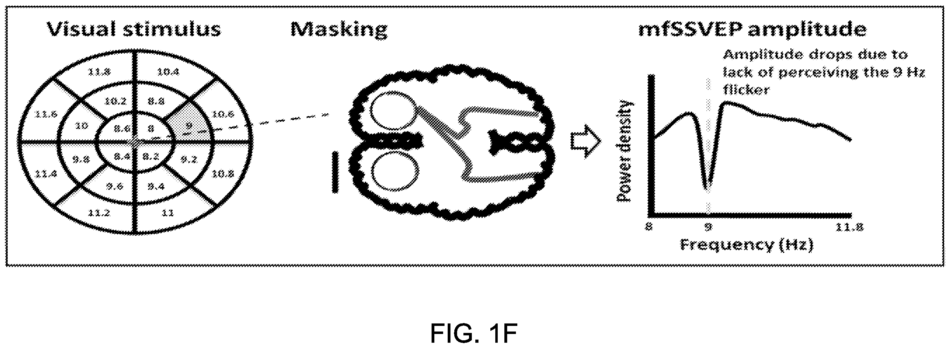

Exemplary implementations were performed using an exemplary mfSSVEP technique for assessment of visual field loss. FIG. 1F shows diagrams depicting an exemplary implementation using multifocal steady-state visual-evoked potentials (mfSSVEP) for assessment of visual field defects. As shown in the diagrams of FIG. 1F, by presenting multiple frequency-tagged flickering (alternating black/white) sectors in the monocular visual field, a sector(s) corresponding to a visual field deficit(s) would be less perceivable, if not totally unperceivable, and thereby would have a weaker SSVEP signal, e.g., compared to the brain responses to other visual stimuli presented at normal visual spots. For example, as illustrated in the diagrams of FIG. 1F, if the visual deficit exists exactly within the 9 Hz visual sector, the 9 Hz SSVEP amplitude tends to deteriorate to some extent compared to other SSVEP frequencies.

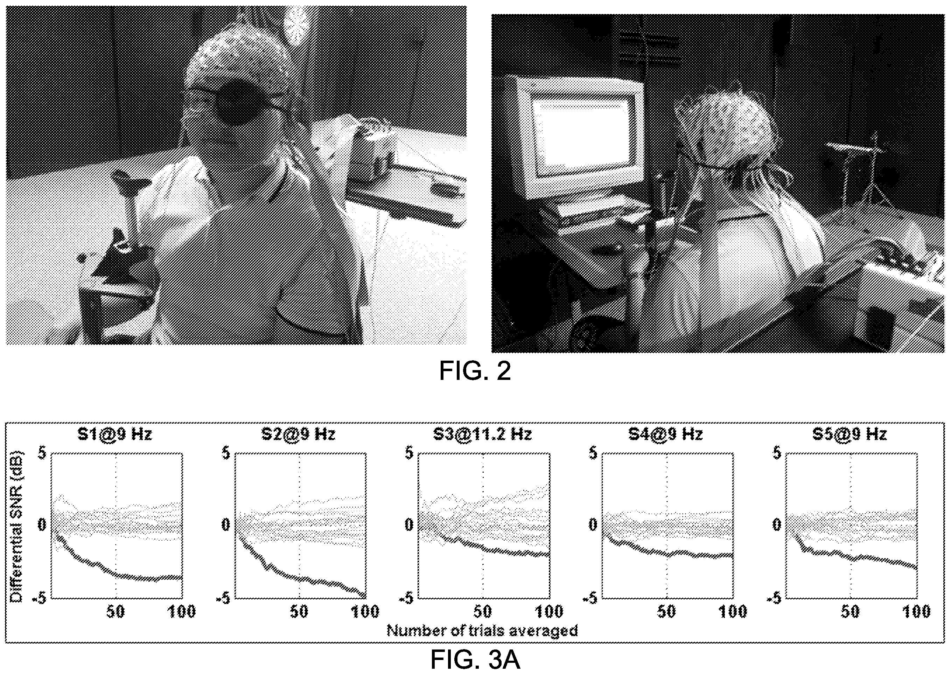

Five healthy participants (e.g., 4 males and 1 female) with normal or corrected-to-normal vision participated in the exemplary implementations. The EEG data were recorded using a 128-channel BioSemi ActiveTwo EEG system (e.g., from Biosemi, Inc.) according to a modified 10-20 international system, as depicted in the images of FIG. 2. FIG. 2 shows images of an exemplary EEG setup used in exemplary implementations of the disclosed mfSSVEP visual field loss analysis techniques. The visual experiment was conducted inside a dark, soundproof shielded room. Visual stimuli were presented on a 19" CRT monitor in front of the participants at a distance of 50 cm with a refresh rate of 140 Hz and a resolution of 800×600 pixels. To test the monocular visual field, the left eye was occluded and the participant was instructed to maintain fixation of the right eye at the center of the mfSSVEP stimulation screen throughout the entire experiment. In one exemplary implementation, for example, the modified 10-20 international system configuration of electrodes included 19 channels of electrodes arranged over parietal and occipital areas such as Pz, PO3, POz, PO4, O1, Oz, and O2. In some implementations, for example, the EEG electrode configurations of the EEG unit 111 can include a single electrode placed on the head or neck of the patient, e.g., such as on head over the occipital region of the brain. For example, the single electrode channel configuration can include 1 channel - Oz. In some implementations, for example, the EEG electrode configuration of the EEG unit 111 can include 2 electrode channels (e.g., 2 channels - O1 and O2); or in other implementations, for example, as few as 3 channels (e.g., 3 channels - O1, O2 and Oz); or in other implementations, for example, as few as 4 channels (e.g., 4 channels - POz, O1, O2 and Oz); or or in other implementations, for example, as few as 8 channels (e.g., 8 channels - PO5, PO3, POz, PO4, PO6, O1, O2 and Oz).

In these exemplary implementations, a layout of visual stimuli was designed to include 20 sectors in three concentric rings (e.g., subtending 6°, 15°, and 25° of the visual field). All sectors flickered concurrently at different frequencies ranging from 8 to 11.8 Hz with a frequency resolution of 0.2 Hz. To test the exemplary mfSSVEP visual field loss analysis technique, a visual field loss (DEFICIT) condition was mimicked by replacing the 9 Hz sector (e.g., the 0-45° patch in the middle ring as shown in the diagram of FIG. 1F) with a black patch, in contrast to the CONTROL condition in which all 20 sectors flickered concurrently. Each participant underwent an experiment including five 4-min sessions with a minute inter-session rest to avoid visual fatigue. Each session repeated the visual sequence of a 1-min CONTROL condition and a 1-min DEFICIT condition twice. Each condition contained at least ten 5-s visual trials interleaved with 1-s rest, and in some implementations, each condition included 100 trials of 5 seconds per trial.

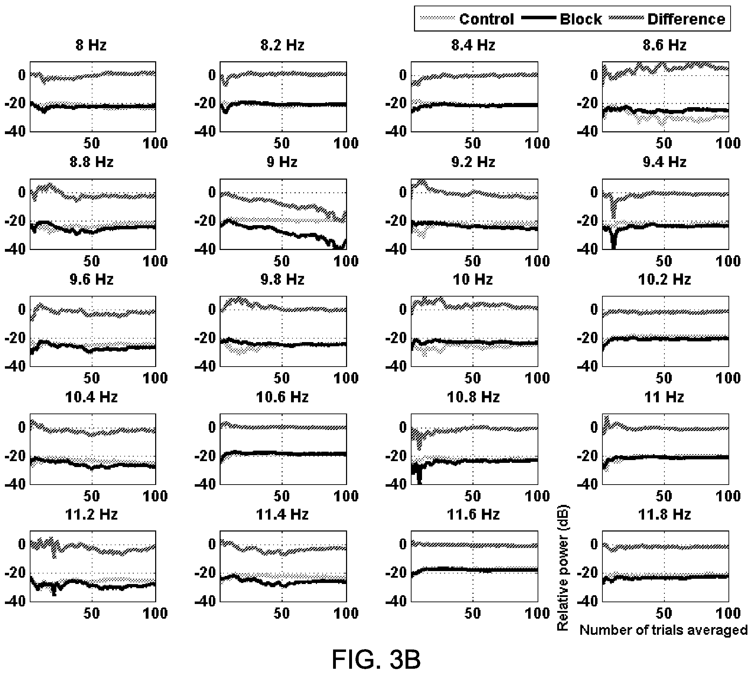

The exemplary dataset for each participant contained 100 5-s DEFICIT and CONTROL trials (e.g., 10 trials × 2 conditions × 5 sessions) for analysis. FIG. 3A shows data plots depicting the CONTROL-DEFICIT comparison for five participants, in which the exemplary red line of the plots represent the SSVEP frequency with the lowest signal-to-noise-ratio versus other frequencies (e.g., represented by the gray lines). FIG. 3B shows data plots depicting a BLOCK-CONTROL comparison in mfSSVEP profiles from a representative subject. The exemplary empirical results showed that four of five participants consistently exhibited a significant deterioration of the 9 Hz ssVEP amplitude in the DEFICIT condition, e.g., as compared to the CONTROL condition. The inconsistency from a participant (S3) was likely attributed to the absence of gaze-attentive fixation to the visual stimulus during the experiment, according to his self-report after the experiment.

These exemplary results demonstrated that visual field deficits mimicked by disabling the 9 Hz sector did result in a significant SSVEP attenuation at the corresponding frequency. The exemplary results of these implementations suggest that the dynamics of mfSSVEP amplitude is capable of serving as an objective biomarker to assess potential visual field deficits.

### Exemplary Embodiments and Implementations of a Portable, Objective mfSSVEP-based Visual Field Assessment Platform of the Disclosed Technology

As shown in the block diagram of FIG. 1A, the disclosed technology includes a portable, objective mfSSVEP-based visual field assessment platform, e.g., which includes and integrates a wearable, wireless dry EEG system and a head-mounted display. The portable devices and systems can acquire and process measurements of reliable mfSSVEP signals that are quantitatively associated with visual field integrity of a subject.

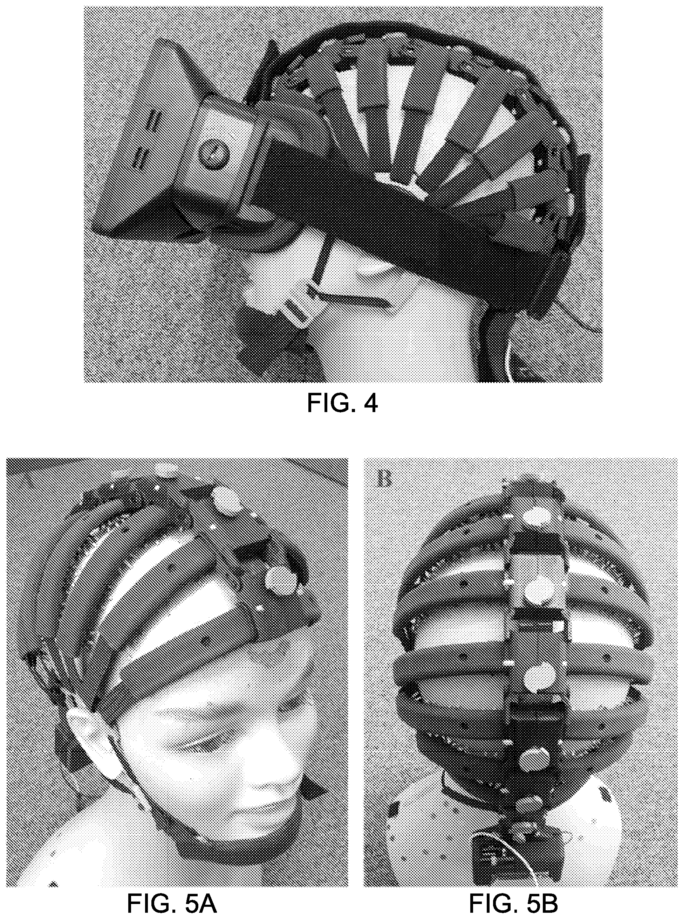

The exemplary platform can quantitate the integrity of monocular visual fields by the characterization of mfSSVEP signal data. For example, by integrating a lightweight, wearable, and wireless multi-channel EEG system and a head-mounted display, the disclosed portable platform can allow assessment of visual field integrity in unconstrained situations and outside clinic environment. FIG. 4 shows an image of an exemplary portable EEG unit 111 of the disclosed portable, objective mfSSVEP-based visual field assessment platform. Exemplary characteristics of the exemplary EEG unit shown in FIG. 4 include easy-to-use and environment-free, which can avoid the burden of setting up laboratory EEG recording, enabling routine visual-field assessment or screening for visual field loss in non-clinic environments. Exemplary implementations using the exemplary portable devices and systems of the disclosed technology demonstrated that the strength of mfSSVEP signals is informative to serve as an objective indicator to reflect possible deficits in a monocular visual field.

In exemplary implementations using the exemplary portable system platform, the following methods were performed. An exemplary wearable, wireless high-density EEG unit of the portable system platform was employed, e.g., featuring dry and non-prep electrodes and wireless telemetry to sample EEG signals at 250 Hz, as depicted in FIGS. 5A and 5B. FIG. 5A shows a side view image and FIG. 5B shows a back view image of an exemplary wearable, wireless 64-channel dry EEG unit. Unlike a conventional and cumbersome EEG experiments, for example, users of the exemplary portable system can easily put on the wearable EEG unit by themselves in their living environments. For example, the exemplary portable system can in some embodiments employ a 64-channel high-density EEG, while in other embodiments can employ a reduced number of channels to provide an optimal configuration or montage for portably detecting mfSSVEP, e.g., outside of a non-office based environment. The exemplary portable system can employ advanced signal processing methods, e.g., such as spatial filtering and artifact removal, to improve signal-to-noise ratio of mfSSVEP from high-density recording.

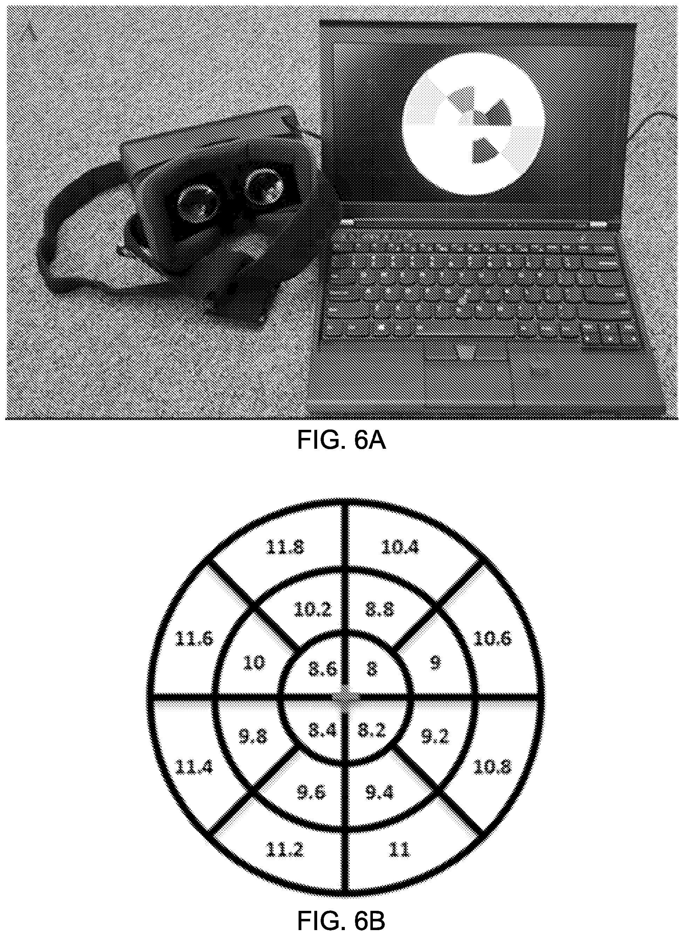

An exemplary head-mounted display unit of the portable system platform utilized an Oculus Rift goggle (Oculus VR, Inc.) to deliver the mfSSVEP stimulation, as depicted in FIGS. 6A and 6B. FIG. 6A shows an image of the exemplary head-mounted display unit to present the mfSSVEP stimulation, and FIG. 6B shows a diagram of the exemplary layout of the mfSSVEP stimulation presentation. The exemplary head-mounted display unit provides an inexpensive solution with a good image resolution of 1280×800, and also provides a whole-eye coverage allowing a controlled environment for visual-field assessment. The mobility can be obtained by communicatively connecting the exemplary head-mounted display unit to one or more mobile communication devices, e.g., such as a laptop as shown in the image of FIG. 6A, or other mobile devices. For example, tablets, smartphones, or wearable computing devices can also be communicatively connected to the exemplary head-mounted display unit. In the exemplary implementations, for example, the same layout of the mfSSVEP stimulation was used as in other exemplary implementations, e.g., including 20 sectors in three rings (e.g., subtending 6°, 15°, and 25° of the visual field) flickering at frequencies from 8 to 11.8 Hz with a frequency resolution of 0.2 Hz were used (as shown in the diagram of FIG. 6B). For example, subjects can be tested under different experimental conditions, by varying testing parameters similarly to those previously described exemplary implementations. For example, a visual field loss condition can be mimicked by using black patches to replace different sectors on the visual field and evaluate mfSSVEP signal loss compared to a control condition.

The example portable system can be used for acquisition of mfSSVEP signals to obtain such data. For example, exemplary techniques such as independent component analysis (ICA) and differential canonical correlation analysis can be implemented successfully for blind source separation and to enhance detectability of SSVEP signals.

### Exemplary EOG-Guided Methods to Assess Eye-Gaze During Testing with the Exemplary Portable Platform

Electrooculogram (EOG) methods of the disclosed technology can be utilized to successfully identify fixation losses and allow identification of unreliable mfSSVEP signals to be removed from further analyses. For example, from the earlier implementations of an mfSSVEP technique for assessment of visual field loss, the strength of mfSSVEP in one of the five participates failed to accurately reflect the mimicked visual deficit, as depicted previously in FIG. 3A. The reason, for example, may be attributed to the absence of proper gaze fixation during the examination based on the patient's self-report. In order to assure matching of SSVEP signals to corresponding visual field locations, subjects need to remain fixating on the central target location during the testing. Due to the short duration of testing trials, this can be achieved in most subjects, yet, the disclosed technology includes a mechanism to identify and exclude unreliable EEG signals produced by fixation losses. This is especially relevant in portable testing that may be performed without supervision.

In some embodiments, for example, the disclosed portable mfSSVEP systems can include an EOG unit. In one example embodiment, the EOG unit can include two or more dry and soft electrodes to be placed proximate the outer canthus of a subject's eyes (e.g., one or more electrodes per eye) to measure corneo-retinal standing potentials, and are in communication with a signal processing and wireless communication unit of the EOG unit to process the acquired signals from the electrodes and relay the processed signals as data to the data processing unit 120 of the portable system 100. In some implementations, the electrodes of the EOG unit can be in communication with the EEG unit 111 or visual display unit 112 to transfer the acquired signals from the outer canthus-placed electrodes of the EOG unit to the data processing unit 120.

For example, in order to remove unreliable EEG signals occurring from fixation losses, the disclosed techniques can concurrently monitor subjects' electrooculogram (EOG) signals to evaluate the gaze fixation. By placing the dry and soft electrodes of the EOG unit to the outer canthus of the eyes, the electric field changes associated with eye movements, e.g., such as blinks and saccades, can be monitored. There is a linear relationship between horizontal and vertical EOG signals and the angle of eye rotation within a limited range (e.g., approximately 30°). This relationship can be used in determining the exact coordinates of eye fixations on a visual display. In some implementations, a calibration sequence can be used at the start of recording to determine the transformation equations. Accordingly, for example, an EOG-guided mfSSVEP analysis can be implemented to automatically exclude the EEG segments where the subjects do not gaze at the center of the stimulation. To record EOG signals, four prefrontal electrodes can be switched to record the EOG signals, e.g., since mfSSVEP signals are presumably weak in the prefrontal regions. In one example in which the EOG unit includes four electrodes, two electrodes can be placed below and above the right eye and another two will be placed at the left and right outer canthus. The EOG unit can be used to assess the accuracy of the portable mfSSVEP system by identifying potentially unreliable EEG signals induced by loss of fixation. For example, the data processing unit 120 can process the acquired signals from the EOG unit electrodes with the EEG data acquired from the EEG unit 111 to identify unreliable signals, which can then be removed from the analysis of visual field integrity. For example, the data processing unit 120 can execute analytical techniques to provide signal source separation. Additionally, or alternatively, for example, the disclosed portable mfSSVEP systems can include an eye tracking unit to monitor losses of fixation, e.g., and can further provide a reference standard. For example, the eye tracking unit can be included, integrated, and/or incorporated into the visual display unit 112 (e.g., exemplary head-mounted display), for example.

### Exemplary Implementations to Evaluate the Reproducibility of Measurements obtained with the Exemplary Portable Platform

Exemplary implementations can be performed to evaluate the reproducibility of measurements obtained with the exemplary portable platform in evaluating its ability to detect visual field loss in patients with glaucoma compared to healthy control subjects. The disclosed portable systems can provide reproducible intra- and inter-visit mfSSVEP signals. mfSSVEP signals in patients with glaucomatous visual field loss will be significantly different than those in healthy control subjects. For example, to have clinical applicability, measurements obtained with portable systems need to be reproducible and be able to detect visual field losses in patients with glaucoma. Good reproducibility is a fundamental requirement in order to allow detection of change over time. If a test is to be used for screening, diagnosis, and/or detection of glaucoma progression, an initial step is demonstrating that a portable system is able to distinguish patients with glaucomatous field loss from healthy individuals.

Exemplary implementations were conducted to demonstrate the reproducibility and diagnostic accuracy studies. Such implementations included participation of a group of 10 healthy and 10 glaucomatous subjects. These subjects had not been tested previously with the system. The subjects underwent five sessions of testing per visit for five different visits, spaced at intervals of approximately 1 week apart. Reproducibility measures were obtained, e.g., including coefficients of variation and intraclass correlation coefficients for the relevant measurements obtained by the portable system. For diagnostic accuracy evaluation, for example, tests of an additional sample of 20 healthy and 20 glaucomatous subjects were performed, e.g., totaling 30 subjects in each group. Glaucomatous subjects were shown to have repeatable abnormal visual field defects on SAP (SITA 24-2). For example, assessment of diagnostic accuracy was performed using receiver operating characteristic (ROC) curves. The sample size for this experiment provided 83% power to detect a minimum difference of 0.25 in ROC curve area compared to chance.

### Examples

The following examples are illustrative of several embodiments of the present technology. Other exemplary embodiments of the present technology may be presented prior to the following listed examples, or after the following listed examples.
In an example of the present technology (example 1), a system for monitoring brain activity associated with visual field of a user includes a sensor unit to acquire electroencephalogram (EEG) signals including one or more electrodes attached to a casing wearable on the head of a user; visual display unit including a display screen to present visual stimuli to the user in a plurality of sectors of a visual field, in which the presented visual stimuli includes an optical flickering effect at a selected frequency mapped to each sector of the visual field, the visual stimuli configured to evoke multifocal steady-state visual-evoked potentials (mfSSVEP) in the EEG signals exhibited by the user acquired by the sensor unit; and a data processing unit in communication with the sensor unit and the visual display unit to analyze the acquired EEG signals and produce an assessment of the user's visual field.
Example 2 includes the system as in example 1, in which the produced assessment of the user's visual field is a quantitative assessment that indicates if there is a presence of a visual field defect in the user's visual field.
Example 3 includes the system as in example 1, in which the one or more electrodes of the sensor unit include dry electrodes operable to acquire the EEG signals without a conductive gel interfaced between the electrodes and the user.
Example 4 includes the system as in example 1, in which the one or more electrodes includes a single electrode channel Oz.
Example 5 includes the system as in example 1, in which the one or more electrodes include a plurality of the electrodes that are arranged at particular locations on the head of the subject according to the international 10-20 system.
Example 6 includes the system as in example 1, in which the system is portable to enable the user to operate the system in the user's living environment and on a routine or continuous basis.
Example 7 includes the system as in example 1, in which the selected frequency of the optical flickering effect for a particular sector is at a different frequency with respect to the optical flickering effect at a proximate sector or with respect to the optical flickering effects in the other sectors of the visual field.
Example 8 includes the system as in example 7, in which the visual stimuli includes multiple and repetitive optical effects flickering at the selected frequency in the corresponding sector of the visual field of the user.
Example 9 includes the system as in example 7, in which the visual stimuli is presented in 20 sectors in three concentric rings including subtending 6°, 15°, and 25° of the visual field.
Example 10 includes the system as in example 7, in which the selected frequencies of the flickerings of the visual stimuli are greater than 6 Hz.
Example 11 includes the system as in example 1, further including an electrooculogram (EOG) unit including one or more electrodes to be placed proximate the outer canthus of each of the user's eyes to measure corneo-retinal standing potential (CRSP) signals, in which the one or more electrodes of the EOG unit are in communication with the data processing unit to process the acquired CRSP signals from the one or more electrodes to determine movements of the user's eyes.
Example 12 includes the system as in example 1, in which the visual display unit is configured to be wearable over the user's eyes for the user to view the presented visual stimuli on the display screen.
Example 13 includes the system as in example 12, further including an eye tracking device including a camera employed in the wearable visual display unit and in communication with the data processing unit, in which the camera is operable to record images of the user's eyes.
In an example of the present technology (example 14), a method for examining a visual field of a subject includes presenting, to a subject, visual stimuli in a plurality of sectors of a visual field of a subject, in which for each sector the presented visual stimuli includes an optical flickering effect at a selected frequency; acquiring electroencephalogram (EEG) signals from one or more electrodes in contact with the head of the subject; processing the acquired EEG signals to extract multifocal steady-state visual-evoked potentials (mfSSVEP) data associated with the subject's EEG signal response to the presented visual stimuli; and producing a quantitative assessment of the visual field of the subject based on the MfSSVEP data.
Example 15 includes the method as in example 14, in which the quantitative assessment provides an indication if there is a presence of a visual field defect in the user's visual field.
Example 16 includes the method as in example 15, in which the producing the quantitative assessment includes determining the presence of the visual field defect in a sector having a mfSSVEP signal below a predetermined threshold.
Example 17 includes the method as in example 14, in which the visual stimuli includes multiple and repetitive optical effects flickering at the selected frequency in the corresponding sector of the visual field of the subject.
Example 18 includes the method as in example 14, in which the one or more electrodes are included in a sensor unit wearable on the subject's head and include a single electrode channel Oz positioned over the occipital region of the head of the subject when the sensor unit is worn by the user.
Example 19 includes the method as in example 14, in which the one or more electrodes are included in a sensor unit wearable on the subject's head such that the electrodes are arranged at particular locations on the sensing unit to be positioned on the head of the subject when the sensor unit is worn by the user.
Example 20 includes the method as in example 14, in which the one or more electrodes include dry electrodes operable to acquire the EEG signals without a conductive gel interfaced between the electrodes and the subject.
Example 21 includes the method as in example 14, further including monitoring movements of the user's eyes to determine instances associated with the user gazing away from the center of the visual field.
Example 22 includes the method as in example 14, in which the monitoring the movements of the user's eyes includes using an electrooculogram (EOG) unit including one or more electrodes placed proximate the outer canthus of each of the user's eyes to measure corneo-retinal standing potential (CRSP) signals.
Example 23 includes the method as in example 14, in which the monitoring the movements of the user's eyes includes using an eye tracking system.
Example 24 includes the method as in example 14, in which the selected frequency of the optical flickering effect for a particular sector is presented at a different frequency with respect to the optical flickering effect at a proximate sector or with respect to the optical flickering effects in the other sectors of the visual field.
Example 25 includes the method as in example 14, further including forming a spatial visual stimulus display having the plurality of the sectors at different spatial locations on the spatial visual stimulus display, in which, for each sector, the sector includes the optical flickering effect that changes at a designated frequency with respect to at least a proximate sector or the other sectors of the visual stimulus display.
Example 26 includes the method as in example 14, in which the producing the quantitative assessment includes analyzing the mfSSVEP data with respect to the designated frequencies that are mapped to the sectors at the different spatial locations on the spatial visual stimulus display, in which the analyzing includes quantitatively comparing an mfSSVEP signal value at a particular frequency of the designated frequencies to a threshold value, and determining the presence of a visual field defect in the sector to which the particular frequency is mapped if the mfSSVEP signal value is less than the threshold value.
In an example of the present technology (example 27), a portable system for monitoring brain activity associated with visual field of a user includes a brain signal sensor device to acquire electroencephalogram (EEG) signals including one or more electrodes attached to a casing wearable on the head of a user; a wearable visual display unit to present visual stimuli to the user and structured to include a display screen and a casing able to secure to the head of the user, in which the wearable visual display is operable to present the visual stimuli in a plurality of sectors of the user's visual field, such that for each sector the presented visual stimuli includes an optical flickering effect at a selected frequency, and in which the visual stimuli are configured to evoke multifocal steady-state visual-evoked potentials (mfSSVEP) in the EEG signals exhibited by the user acquired by the brain signal sensor device; a data processing unit in communication with the brain signal sensor device and the wearable visual display unit to provide the visual stimuli to the wearable visual display unit and to analyze the acquired EEG signals and produce an assessment of the user's visual field; and an electrooculogram (EOG) unit including one or more electrodes to be placed proximate the outer canthus of each of the user's eyes to measure corneo-retinal standing potential (CRSP) signals, in which the one or more electrodes of the EOG unit are in communication with the data processing unit to process the acquired CRSP signals from the one or more electrodes to determine movements of the user's eyes.
Example 28 includes the system as in example 27, in which the data processing unit is included in a computing device including a laptop or desktop computer; a mobile communication device including a smartphone, a tablet, or a wearable computing device; or a network computer system.
Example 29 includes the system as in example 27, in which the produced assessment of the user's visual field is a quantitative assessment that indicates if there is a presence of a visual field defect in the user's visual field.
Example 30 includes the system as in example 27, in which the one or more electrodes of the brain signal sensor device include dry electrodes operable to acquire the EEG signals without a conductive gel interfaced between the electrodes and the user.
Example 31 includes the system as in example 27, in which the one or more electrodes includes a single electrode channel Oz.
Example 32 includes the system as in example 27, further including an eye tracking device including a camera employed in the wearable visual display unit and in communication with the data processing unit, in which the camera is operable to record images of the user's eyes.
Example 33 includes the system as in example 27, in which the visual stimuli includes multiple and repetitive optical effects flickering at the selected frequency in the corresponding sector of the visual field of the user, and in which the selected frequencies of the flickerings of the visual stimuli are greater than 6 Hz.
Example 34 includes the system as in example 27, in which the wearable visual display unit is operable to form a spatial visual stimulus display on the display screen having the plurality of the sectors at different spatial locations on the spatial visual stimulus display, in which, for each sector, the sector includes the optical flickering effect that changes at a designated frequency with respect to at least a proximate sector or the other sectors of the visual stimulus display.
Example 35 includes the system as in example 34, in which the data processing unit is operable to produce the quantitative assessment by analyzing the mfSSVEP data with respect to the designated frequencies that are mapped to the sectors at the different spatial locations on the spatial visual stimulus display, in which the analyzing includes quantitatively comparing an mfSSVEP signal value at a particular frequency of the designated frequencies to a threshold value, and determining the presence of a visual field defect in the sector to which the particular frequency is mapped if the mfSSVEP signal value is less than the threshold value.

Implementations of the subject matter and the functional operations described in this patent document can be implemented in various systems, digital electronic circuitry, or in computer software, firmware, or hardware, including the structures disclosed in this specification and their structural equivalents, or in combinations of one or more of them. Implementations of the subject matter described in this specification can be implemented as one or more computer program products, i.e., one or more modules of computer program instructions encoded on a tangible and non-transitory computer readable medium for execution by, or to control the operation of, data processing apparatus. The computer readable medium can be a machine-readable storage device, a machine-readable storage substrate, a memory device, a composition of matter effecting a machine-readable propagated signal, or a combination of one or more of them. The term "data processing apparatus" encompasses all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, or a combination of one or more of them.

A computer program (also known as a program, software, software application, script, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Computer readable media suitable for storing computer program instructions and data include all forms of nonvolatile memory, media and memory devices, including by way of example semiconductor memory devices, e.g., EPROM, EEPROM, and flash memory devices. The processor and the memory can be supplemented by, or incorporated in, special purpose logic circuitry.

While this patent document contains many specifics, these should not be construed as limitations on the scope of any invention as defined by the claims.

Similarly, while operations are depicted in the drawings in a particular order, this should not be understood as requiring that such operations be performed in the particular order shown or in sequential order, or that all illustrated operations be performed, to achieve desirable results. Moreover, the separation of various system components in the embodiments described in this patent document should not be understood as requiring such separation in all embodiments.

Only a few implementations and examples are described and other implementations, enhancements and variations can be made based on what is described and illustrated in this patent document.

## Claims

1. A portable head-mounted display system (100) for monitoring brain activity associated with the visual field of a user, comprising:
a casing wearable on the head of the user;
a sensor unit (111) configured to acquire electroencephalogram (EEG) signals including one or more dry electrodes attached to the casing wearable on the head of a user;
a visual display unit (112) including a display screen configured to present visual stimuli to the user in a plurality of sectors of a visual field, wherein the visual stimuli include a plurality of regions arranged in concentric rings, wherein each of the plurality of regions includes a multiple and repetitive optical flickering effect concurrently with the other regions and at a selected frequency different from the flickering frequency of the other regions, wherein the optical flickering effect at the selected frequency for the region is mapped to each sector of the visual field, and wherein the visual stimuli are configured to evoke multifocal steady-state visual-evoked potentials (mfSSVEP) in the EEG signals exhibited by the user acquired by the sensor unit; and
a data processing unit (120) configured to:
generate the visual stimuli;
communicate with the sensor unit and the visual display unit;
analyze the acquired EEG signals; and
produce an assessment of the visual field of the user,
wherein the visual display unit provides whole-eye coverage and a controlled visual environment for the assessment.

2. The system of claim 1, wherein the produced assessment of the visual field of the user is a quantitative assessment that indicates if there is a presence of a visual field defect in the visual field of the user.

3. The system of claim 1, wherein the one or more electrodes of the sensor unit include:
dry electrodes operable to acquire the EEG signals without a conductive gel interfaced between the electrodes and the user,
a single electrode channel Oz, or
a plurality of the electrodes that are arranged at particular locations on the head of the user according to the international 10-20 system.

4. The system of claim 1, wherein the system is portable to enable the user to operate the system in the user's living environment and on a routine or continuous basis.

5. The system of claim 1, wherein the selected frequency of the optical flickering effect for a particular region is at a different frequency with respect to the optical flickering effect at a proximate region or with respect to the optical flickering effects in the other regions of the visual stimuli.

6. The system of claim 5, wherein the visual stimuli are presented in 20 regions in three concentric rings including subtending 6°, 15°, and 25° of the visual field.

7. The system of claim 5, wherein the selected frequencies of the optical flickering effect of each of the plurality of regions of the visual stimuli are greater than 6 Hz.

8. The system of claim 1, further comprising:
an electrooculogram (EOG) unit including one or more electrodes to be placed proximate the outer canthus of each of the user's eyes to measure corneo-retinal standing potential (CRSP) signals, wherein the one or more electrodes of the EOG unit are in communication with the data processing unit to process the acquired CRSP signals from the one or more electrodes to determine movements of the user's eyes.

9. The system of claim 1, wherein the visual display unit (112) is configured to be wearable over the user's eyes for the user to view the presented visual stimuli on the display screen, and the system further comprises:
an eye tracking device including a camera employed in the wearable visual display unit and in communication with the data processing unit, wherein the camera is operable to record images of the user's eyes.

10. A method (180) for examining a visual field of a subject using a portable head-mounted display system, the method comprising:
generating a visual stimuli;
presenting (182), to the subject in a controlled visual environment that provides whole-eye coverage using a visual display unit, the visual stimuli in a plurality of sectors of a visual field of the subject, wherein the visual stimuli include a plurality of regions arranged in concentric rings, wherein each of the plurality of regions includes a multiple, continuous and repetitive optical flickering effect concurrently with the other regions and at a selected frequency different from the flickering frequency of the other regions, and wherein the optical flickering effect at the selected frequency for the region is mapped to each sector of the visual field;
acquiring (184) electroencephalogram (EEG) signals from one or more dry electrodes that are included in a sensor unit wearable on the subject's head and in contact with the head of the subject;
processing (186) the acquired EEG signals to extract multifocal steady-state visual-evoked potentials (mfSSVEP) data associated with the subject's EEG signal response to the presented visual stimuli; and producing (188) a quantitative assessment of the visual field of the subject based on the MfSSVEP data.

11. The method of claim 10, wherein the visual stimuli are presented in 20 regions in three concentric rings including subtending 6°, 15°, and 25° of the visual field.

12. The method of claim 10, wherein the one or more electrodes include a single electrode channel Oz positioned over the occipital region of the head of the subject when the sensor unit is worn by the subject, or
the electrodes include dry electrodes operable to acquire the EEG signals without a conductive gel interfaced between the electrodes and the subject.

13. The method of claim 10, wherein the monitoring the movements of the subject's eyes includes using an electrooculogram (EOG) unit including one or more electrodes placed proximate the outer canthus of each of the subject's eyes to measure corneo-retinal standing potential (CRSP) signals.

14. The method of claim 10, further comprising:
forming a spatial visual stimulus display having the plurality of the regions at different spatial locations on the spatial visual stimulus display, wherein, for each region, the region includes the optical flickering effect that changes at a designated frequency with respect to at least a proximate region or the other regions of the visual stimulus display.

## Patentansprüche

1. Tragbares Datenhelmsystem (100) zum Überwachen von Gehirnaktivität, die mit dem Sichtfeld eines Benutzers verknüpft ist, das Folgendes umfasst:
ein Gehäuse, das auf dem Kopf des Benutzers tragbar ist;
eine Sensoreinheit (111), die konfiguriert ist, um Elektroenzephalogramm(EEG)-Signale zu erfassen, einschließlich einer oder mehrerer Trockenelektroden, die an das Gehäuse angebracht sind, das auf dem Kopf eines Benutzers tragbar ist;
eine visuelle Anzeigeeinheit (112), die einen Anzeigebildschirm einschließt, der konfiguriert ist, um dem Benutzer visuelle Reize in mehreren Sektoren eines Sichtfelds zu präsentieren, wobei die visuellen Reize mehrere Regionen einschließen, die in konzentrischen Ringen angeordnet sind, wobei jede der mehreren Regionen einen mehrfachen und sich wiederholenden optischen Flackereffekt gleichzeitig mit den anderen Regionen und bei einer ausgewählten Frequenz, die sich von der Flackerfrequenz der anderen Regionen unterscheidet, einschließt, wobei der optische Flackereffekt bei der ausgewählten Frequenz für die Region jedem Sektor des Sichtfelds zugeordnet wird, und wobei die visuellen Reize konfiguriert sind, um multifokale visuell evozierte Potentiale in einem stabilen Zustand (*multifocal steady-state visual-evoked potentials* - mfSSVEP) in den EEG-Signalen zu evozieren, die durch den Benutzer vorgewiesen werden, die durch die Sensoreinheit erfasst werden; und
eine Datenverarbeitungseinheit (120), die für Folgendes konfiguriert ist:
Erzeugen der visuellen Reize;
Kommunizieren mit der Sensoreinheit und der visuellen Anzeigeeinheit;
Analysieren der erfassten EEG-Signale; und
Erstellen einer Beurteilung des Sichtfelds des Benutzers, wobei die visuelle Anzeigeeinheit eine Abdeckung des gesamten Auges und ein kontrolliertes visuelles Umfeld für die Beurteilung bereitstellt.

2. System nach Anspruch 1, wobei die erstellte Beurteilung des Sichtfelds des Benutzers eine quantitative Beurteilung ist, die angibt, ob ein Sichtfelddefekt in dem Sichtfeld des Benutzers vorhanden ist.

3. System nach Anspruch 1, wobei die eine oder die mehreren Elektroden der Sensoreinheit Folgendes einschließen:
Trockenelektroden, die betriebsfähig sind, um die EEG-Signale ohne ein leitfähiges Gel, das in einer Schnittstelle zwischen den Elektroden und dem Benutzer liegt, zu erfassen, ein einzelner Elektrodenkanal Oz oder mehrere der Elektroden, die gemäß des internationalen 10-20-Systems an speziellen Stellen auf dem Kopf des Benutzers angeordnet sind.

4. System nach Anspruch 1, wobei das System tragbar ist, um es dem Benutzer zu ermöglichen, das System in dem Lebensumfeld des Benutzers und routinemäßig oder fortlaufend zu betreiben.

5. System nach Anspruch 1, wobei die ausgewählte Frequenz des optischen Flackereffekts für eine spezielle Region bei einer unterschiedlichen Frequenz hinsichtlich des optischen Flackereffekts an einer nahen Region oder hinsichtlich der visuellen Flackereffekte in den anderen Regionen der visuellen Reize liegt.

6. System nach Anspruch 5, wobei die visuellen Reize in 20 Regionen in drei konzentrischen Ringen präsentiert werden, einschließlich eines Abschneidens von 6°, 15° und 25° des Sichtfeldes.

7. System nach Anspruch 5, wobei die ausgewählten Frequenzen des optischen Flackereffekts von jedem der mehreren Regionen der visuellen Reize über 6 Hz liegen.

8. System nach Anspruch 1, das ferner Folgendes umfasst:
eine Elektrookulogramm(EOG)-Einheit einschließlich einer oder mehrerer Elektroden, die nahe des äußeren Kanthus jedes Auges des Benutzers zu platzieren sind, um korneoretinale Bestandspotential(*corneo-retinal standing potential* - CRSP)-Signale zu messen, wobei die eine oder die mehreren Elektroden der EOG-Einheit mit der Datenverarbeitungseinheit in Kommunikation stehen, um die erfassten CRSP-Signale von der einen oder den mehreren Elektroden zu verarbeiten, um Bewegungen der Augen des Benutzers zu bestimmen.

9. System nach Anspruch 1, wobei die visuelle Anzeigeeinheit (112) konfiguriert ist, um über den Augen des Benutzers tragbar zu sein, damit der Benutzer die präsentierten visuellen Reize auf dem Anzeigebildschirm sehen kann, und das System ferner Folgendes umfasst:
eine Blickbewegungsregistrierungsvorrichtung einschließlich einer Kamera, die in der tragbaren visuellen Anzeigeeinheit eingesetzt wird und in Kommunikation mit der Datenverarbeitungseinheit steht, wobei die Kamera betriebsfähig ist, um Bilder der Augen des Benutzers aufzuzeichnen.

10. Verfahren (180) zum Untersuchen eines Sichtfeldes eines Subjekts unter Verwendung eines Datenhelmsystems, wobei das Verfahren Folgendes umfasst:
Erzeugen visueller Reize;
Präsentieren (182), dem Subjekt in einem kontrollierten visuellen Umfeld, das die Abdeckung des gesamten Auges unter Verwendung einer visuellen Anzeigeeinheit bereitstellt, der visuellen Reize in mehreren Sektoren eines Sichtfeldes des Subjekts, wobei die visuellen Reize mehrere Regionen einschließen, die in konzentrischen Ringen angeordnet sind, wobei jede der mehreren Regionen einen mehrfachen, fortlaufenden und sich wiederholenden optischen Flackereffekt gleichzeitig mit den anderen Regionen und bei einer ausgewählten Frequenz, die sich von der Flackerfrequenz der anderen Regionen unterscheidet, einschließt, und wobei der optische Flackereffekt bei der ausgewählten Frequenz für die Region jedem Sektor des Sichtfelds zugeordnet wird;
Erfassen (184) von Elektroenzephalogramm(EEG)-Signalen von einer oder mehreren Trockenelektroden, die in einer Sensoreinheit eingeschlossen sind, die auf dem Kopf des Subjekts tragbar ist und mit dem Kopf des Subjekts in Berührung ist;
Verarbeiten (186) der erfassten EEG-Signale, um Daten multifokaler visuell evozierter Potentiale in einem stabilen Zustand (mfSSVEP) zu extrahieren, die mit der EEG-Signalantwort des Subjekts auf die präsentierten visuellen Reize verknüpft sind; und
Erstellen (188) einer quantitativen Beurteilung des Sichtfeldes des Subjekts basierend auf den MfSSVEP-Daten.

11. Verfahren nach Anspruch 10, wobei die visuellen Reize in 20 Regionen in drei konzentrischen Ringen präsentiert werden, einschließlich des Abschneidens von 6°, 15° und 25° des Sichtfeldes.

12. Verfahren nach Anspruch 10, wobei die eine oder die mehreren Elektroden einen einzelnen Elektrodenkanal Oz einschließen, der über der okzipitalen Region des Kopfes des Subjekts positioniert ist, wenn die Sensoreinheit von dem Subjekt getragen wird, oder die Elektroden Trockenelektroden einschließen, die betriebsfähig sind, um die EEG-Signale ohne leitfähiges Gel, das in einer Schnittstelle zwischen den Elektroden und dem Subjekt liegt, zu erfassen.

13. Verfahren nach Anspruch 10, wobei das Überwachen der Bewegungen der Augen des Subjekts ein Verwenden einer Elektrookulogramm(EOG)-Einheit einschließt, einschließlich einer oder mehrerer Elektroden, die nahe des äußeren Kanthus jedes Auges des Subjekts platziert sind, um korneoretinale Bestandspotential(CRSP)-Signale zu messen.

14. Verfahren nach Anspruch 10, das ferner Folgendes umfasst:
Ausbilden einer Anzeige für räumlichen visuellen Reiz, die die mehreren der Regionen an unterschiedlichen räumlichen Stellen auf der Anzeige für räumlichen visuellen Reiz aufweist, wobei, für jede Region, die Region den optischen Flackereffekt einschließt, der sich bei einer vorgesehenen Frequenz hinsichtlich wenigstens einer nahen Region oder der anderen Regionen der visuellen Reizanzeige ändert.

## Revendications

1. Système d'affichage portatif monté sur la tête (100) destiné à surveiller l'activité cérébrale associée au champ visuel d'un utilisateur, comprenant :
un boîtier portable sur la tête de l'utilisateur ;
une unité de capteur (111) configurée pour acquérir des signaux d'électroencéphalogramme (EEG) comportant une ou plusieurs électrodes sèches fixées au boîtier pouvant être portées sur la tête d'un utilisateur ;
une unité d'affichage visuel (112) comportant un écran d'affichage configuré pour présenter des stimuli visuels à l'utilisateur dans une pluralité de secteurs d'un champ visuel, dans lequel les stimuli visuels comportent une pluralité de régions agencées en anneaux concentriques, dans lequel chacune de la pluralité de régions comporte un effet de scintillement optique multiple et répétitif en même temps que les autres régions et à une fréquence sélectionnée 1 différente de la fréquence de scintillement des autres régions, dans lequel l'effet de scintillement optique à la fréquence sélectionnée pour la région est mappé à chaque secteur du champ visuel, et dans lequel les stimuli visuels sont configurés pour évoquer des potentiels évoqués visuels stationnaires multifocaux (mfSSVEP) dans les signaux EEG présentés par l'utilisateur acquis par l'unité de capteur ; et
une unité de traitement de données (120) configurée pour :
générer les stimuli visuels ;
communiquer avec l'unité de capteur et l'unité d'affichage visuel ;
analyser les signaux EEG acquis ; et
produire une évaluation du champ visuel de l'utilisateur,
dans lequel l'unité d'affichage visuel fournit une couverture complète de l'œil et un environnement visuel contrôlé pour l'évaluation.

2. Système selon la revendication 1, dans lequel l'évaluation produite du champ visuel de l'utilisateur est une évaluation quantitative qui indique s'il y a présence d'un défaut de champ visuel dans le champ visuel de l'utilisateur.

3. Système selon la revendication 1, dans lequel la ou les électrodes de l'unité de capteur comportent :
des électrodes sèches utilisables pour acquérir les signaux EEG sans gel conducteur interfacé entre les électrodes et l'utilisateur,
un canal d'électrode Oz unique, ou
une pluralité d'électrodes qui sont disposées à des emplacements particuliers sur la tête de l'utilisateur selon le système international 10-20.

4. Système selon la revendication 1, dans lequel le système est portable pour permettre à l'utilisateur de faire fonctionner le système dans l'environnement de vie de l'utilisateur et sur une base de routine ou continue.

5. Système selon la revendication 1, dans lequel la fréquence sélectionnée de l'effet de scintillement optique pour une région particulière est à une fréquence différente par rapport à l'effet de scintillement optique dans une région proche ou par rapport aux effets de scintillement optique dans les autres régions des stimuli visuels.

6. Système selon la revendication 5, dans lequel les stimuli visuels sont présentés dans 20 régions en trois anneaux concentriques comportant des angles de 6°, 15° et 25° sous-tendant du champ visuel.

7. Système selon la revendication 5, dans lequel les fréquences sélectionnées de l'effet de scintillement optique de chacune de la pluralité de régions des stimuli visuels sont supérieures à 6 Hz.

8. Système selon la revendication 1, comprenant en outre :
une unité d'électrooculogramme (EOG) comportant une ou plusieurs électrodes à placer à proximité du canthus externe de chacun des yeux de l'utilisateur pour mesurer les signaux de potentiel permanent cornéo-rétinien (CRSP), dans lequel la ou les électrodes de l'unité EOG sont en communication avec l'unité de traitement de données pour traiter les signaux CRSP acquis à partir de la ou des électrodes pour déterminer les mouvements des yeux de l'utilisateur.

9. Système selon la revendication 1, dans lequel l'unité d'affichage visuel (112) est configurée pour être portable sur les yeux de l'utilisateur pour que l'utilisateur puisse voir les stimuli visuels présentés sur l'écran d'affichage, et le système comprend en outre :
un dispositif de suivi oculaire comportant une caméra utilisée dans l'unité d'affichage visuel portable et en communication avec l'unité de traitement de données, dans lequel la caméra peut fonctionner pour enregistrer des images des yeux de l'utilisateur.

10. Procédé (180) destiné à examiner un champ visuel d'un sujet à l'aide d'un système d'affichage portatif monté sur la tête, le procédé comprenant :
la génération d'un stimulus visuel ;
la présentation (182), au sujet dans un environnement visuel contrôlé qui fournit une couverture de l'œil entier à l'aide d'une unité d'affichage visuel, les stimuli visuels dans une pluralité de secteurs d'un champ visuel du sujet, les stimuli visuels comportant une pluralité de régions disposés en anneaux concentriques, dans lequel chacune de la pluralité de régions comporte un effet de scintillement optique multiple, continu et répétitif en même temps que les autres régions et à une fréquence sélectionnée différente de la fréquence de scintillement des autres régions, et dans lequel l'effet de scintillement optique au niveau du la fréquence sélectionnée pour la région est mappée sur chaque secteur du champ visuel ;
l'acquisition (184) des signaux d'électroencéphalogramme (EEG) à partir d'une ou de plusieurs électrodes sèches qui sont incluses dans une unité de capteur pouvant être portée sur la tête du sujet et en contact avec la tête du sujet ;
le traitement (186) des signaux EEG acquis pour extraire des données de potentiels évoqués visuels stationnaires multifocaux (mfSSVEP) associées à la réponse du signal EEG du sujet aux stimuli visuels présentés ; et la production (188) d'une évaluation quantitative du champ visuel du sujet à partir des données mfSSVEP.

11. Procédé selon la revendication 10, dans lequel les stimuli visuels sont présentés dans 20 régions en trois anneaux concentriques comportant des angles de 6°, 15° et 25° sous-tendant du champ visuel.

12. Procédé selon la revendication 10, dans lequel la ou les électrodes comportent un canal d'électrode Oz unique positionné sur la région occipitale de la tête du sujet lorsque l'unité de capteur est portée par le sujet, ou les électrodes comportent des électrodes sèches pouvant fonctionner pour acquérir les signaux EEG sans gel conducteur interfacé entre les électrodes et le sujet.

13. Procédé selon la revendication 10, dans lequel la surveillance des mouvements des yeux du sujet comporte l'utilisation d'une unité d'électrooculogramme (EOG) comportant une ou plusieurs électrodes placées à proximité du canthus externe de chacun des yeux du sujet pour mesurer les signaux de potentiel permanent cornéo-rétinien (CRSP).

14. Procédé selon la revendication 10, comprenant en outre :
la formation d'un affichage de stimulus visuel spatial ayant la pluralité des régions à différents emplacements spatiaux sur l'affichage de stimulus visuel spatial, dans lequel, pour chaque région, la région comporte l'effet de scintillement optique qui change à une fréquence désignée par rapport à au moins une région proche ou les autres régions de l'affichage du stimulus visuel.
